# EUROPEAN PATENT SPECIFICATION

(11) **EP 1 302 184 B1**
(45) Date of publication and mention of the grant of the patent: **27.07.2005**
(21) Application number: 02022636.1
(22) Date of filing: 09.10.2002
(51) Int. Cl.: A61F 5/01

(54) **Joint brace for the control and the regulation of the knee joint bending**
Gelenkschiene zur Steuerung und Regelung der Beugung des Kniegelenks
Attelle pour le contrôle et régulation de la flexion du genou

(30) Priority: 10.10.2001 IT VR20010105
(43) Date of publication of application: 16.04.2003
(73) Proprietor: F.G.P. SRL, 37062 Dossobuono (IT)
(72) Inventor: Turrini, Alberto, 37060 Castel d'Azzano (VR) (IT); Ferrigolo, Moreno, 37062 Dossobuono (VR) (IT)
(74) Representative: Petraz, Gilberto Luigi

(56) References cited:
- WO-A-00/18337
- WO-A-01/21114
- WO-A-01/45600
- WO-A-97/40789
- US-A- 5 400 806

## Description

### TECHNICAL FIELD

This invention refers to an orthesis for the control and adjustment of the angulation of the knee joint, in particular to obtain a compensating action in cases of varus or valgus deformity of the knee, in cases of osteoarthritis and in traumatic knee disorders.

More in particular, this invention aims to provide an easily adjustable orthesis which, thanks to the presence of elastically deformable side uprights, provides an appropriately controlled force in correspondence with the knee joint which is sufficient to exercise a pressure that alters the contact between the opposite condyles of the knee bone, decreasing the pressure on them.

This invention can be applied in the medical industry with particular reference to manufacturers of prostheses and braces.

### BACKGROUND ART

It is a known fact that subjects with varus or valgus deformity of the knee can, especially when elderly, present deterioration of the knee joint cartilage.

This deterioration can result in acute or chronic inflammations of the joints or more or less severe forms of osteoarthritis, accompanied by pain which tends to limit the already impaired gait.

Knee joint braces usually consist of a rigid frame in order to ensure adequate support aimed at preventing the onset of strain on the joint and at limiting or preventing torsion during walking.

The frame of classic knee braces comprises means of constraint of the femur and the tibia at points proximal to the knee and a structure connecting these means with a hinge positioned at the knee itself.

To ensure adequate freedom of movement of the limb, the frame develops mostly along the sides of the knee in order to allow reciprocal oscillation between the femur and the tibia.

The means of constraint usually consist of straps fitted with Velcro® which is particularly suitable for adjusting the gripping tension; these straps encircle both the user's femur and tibia.

In the case of subjects with varus or valgus deformity of the knee, the structures designed for a normal alignment of the joint are frequently unable to provide satisfactory protection and the appropriate therapeutic effect; they are also not very comfortable to wear.

A structure designed specifically for this type of disorder must be able to envelop the joint following the individual shape of the limb.

The use of structures that are made according to the requirements of the individual patient has often been a solution but cannot be proposed on an industrial level.

Other solutions foresee the use of particular flexible hinges, inserted in the side arms of the structure and making it possible to adjust the brace on the joint, whether the deformity of the knee is varus or valgus.

One of the aims of these braces is to alleviate the medial or lateral pain caused by osteoarthritis and in particular they attempt to provide a thrust which opposes that caused by the deformity, by applying pressure which alters the contact between the opposite condyles of the knee bone, decreasing the pressure on them and limiting the pain.

The document US-A-5,400,806 presents a brace that comprises a structure with a hinge that works at an angle in order to apply force to the joint.

In this structure, screws are used to adjust the angle of each hinge and consequently the force applied to the joint. The correct positioning of the arm of the structure and the correct force to be applied to the joint do not constitute a very practical operation and it cannot be performed independently and quickly by the person wearing the brace.

The document WO-A-97/40789 presents a brace with angular compensation hinges that can be adjusted directly by the wearer. Adjustment of the hinges modifies the thrust force at the level of the joint.

This brace presents a series of technical disadvantages: first of all the angular force exercised by the hinges fitted to the structure directly affects the central joint which allows the movement of the femur with respect to the tibia, creating obvious disadvantages from a mechanical point of view.

In addition to this, the upright is completely rigid and does not allow any type of adaptation of the structure of the brace to the normal variations in size of the knee joint or to circumstances of irregular tension or strain such as during sports activities for example.

This drawback is even more accentuated when the structure of the brace presents another rigid side upright opposite to the hinged one.

Document WO 01/21114 discloses a brace according to the preamble of claim 1.

The background art also includes a brace which presents both hinges with angular micrometric adjustment and a system which connects the uprights to the central joint and allows a small movement of the joint in the normal direction of the upright axis.

In this way the joint can adapt on one hand to the force exercised by the hinges, causing a lateral thrust on the knee joint, and on the other hand the opposite upright can adapt to the knee joint and the modification of its axis.

In order to allow this movement, the system connecting the uprights inserted in the central joint results in a considerable increase in the size of the joint at the sides of the brace structure. This is particularly unfavourable when the curvature of the limbs is already particularly accentuated and can even be a problem in the case, for example, of valgus deformity of the knee.

The system connecting the uprights to the central joint also has a very delicate structure which, nevertheless, takes most of the force exercised on the joint.

The production of this brace requires additional technical effort for its industrialisation which is not very favourable from an economic point of view.

Finally, the uprights are rigid and cannot be adapted to the anatomical structure of the limb and of the joint, nor can they be modified in the flexion movement between the femur and the tibia, compressing the knee and consequently limiting the fluidity of the movement and safeness in walking.

A similar knee brace is described in document WO 01/45600.

### DESCRIPTION OF THE INVENTION

The aim of the present invention is to provide a knee brace which eliminates or significantly reduces the drawbacks described above.

This invention also aims to provide a brace which can be easily adjusted, even also by the user, and which thanks to the presence of elastically deformable side uprights provides an appropriately controlled force in correspondence with the joint which is sufficient to exercise a pressure that alters the contact between the opposite condyles of the knee bone, decreasing the pressure on them and limiting the pain.

This brace can be advantageously used, for example, in cases of osteoarthritis of the knee.

An additional aim of this invention is to provide a safe and reliable brace in order not to injure the subject wearing it.

This is achieved by means of a knee brace with the features described in the main claim and which can be applied in cases of varus or valgus deformity of the knee, in particular in osteoarthritic disorders of the knee, and in all traumatic or pathological situations requiring compensation action.

The dependent claims describe advantageous embodiments of the invention.

The knee brace according to this invention comprises a fixed frame equipped with joints in the central part and means of restraining the frame to the upper and lower parts of the leg.

This frame consists of at least one upper arch and one lower arch integral with at least one elastically deformable side upright equipped with a joint.

According to this invention the joint consists of two toothed wheel portions designed to engage with each other and each forming a single body with, respectively, the upper and lower parts of the upright.

These toothed wheel portions rotate on two pins and are kept in place by two metal plates fitted on opposite sides.

In this way the joint is rigid, not allowing any movement of its components in the normal direction of the axis of the brace.

According to this invention at least one of the elastically deformable side uprights is equipped with hinges with angular micrometric adjustment, for example hinges of the type forming the subject of the international patent application no. WO01/21114 in the name of the present applicant, which allow fine adjustment of the pressure on the knee joint in cooperation with at least one part, upper or lower with respect to the rigid joint, of the elastically deformable upright.

This pressure is exercised on the knee joint at the level of the brace joint on a condyle plate connected to it and in contact with the knee.

According to this invention, the elasticity of the upper or lower parts of the uprights, with respect to the rigid joint, is achieved by inserting elastic and/or flexible elements which integrate to form a single body with these parts and which can consist of steel foils or composite plastic segments.

Furthermore, according to this invention the upper or lower part, with respect to the joint, of the side upright of the frame opposite the upright exercising the transversal force, obtained by the action of the angular micrometric hinges, is in turn elastic, for example by means of the insertion of appropriate elements. This allows the frame to adapt perfectly to the movements and to the anatomical shape of the limb, thus making the entire structure advantageously elastic and perfectly adaptable to the conformation of the limb, providing a high degree of stability and efficient control of the knee.

The central joints of the side uprights are also, according to this invention, particularly simple and sturdy and, in spite of carrying out the main action of limiting and controlling the knee joint, they are supported by the calibrated flexibility of the upper and lower parts of the side uprights.

### DESCRIPTION OF THE DRAWINGS

Other features and advantages of the invention will become apparent from the following description of an embodiment of the invention, provided purely as a non-restricting example, with reference to the accompanying drawings, in which:
- figure 1 shows a front view of one of the elastically deformable side uprights of a brace according to the present invention, presenting angular micrometric hinges and elastic elements;
- figure 2 shows a front view of one of the elastically deformable side uprights of a brace according to the preseent invention, which can be associated with the upright shown in figure 1 and presenting only the elastic elements; and
- figure 3 presents a perspective view slightly from above of the brace according to this invention.

### DESCRIPTION OF ONE EMBODIMENT

In the figures, the reference number 10 generally indicates a knee brace, in the case in question an orthesis or brace 10 which can be applied in traumatic chronic disorders of the knee, in cases of varus or valgus deformity of the knee and in cases of osteoarthritis.

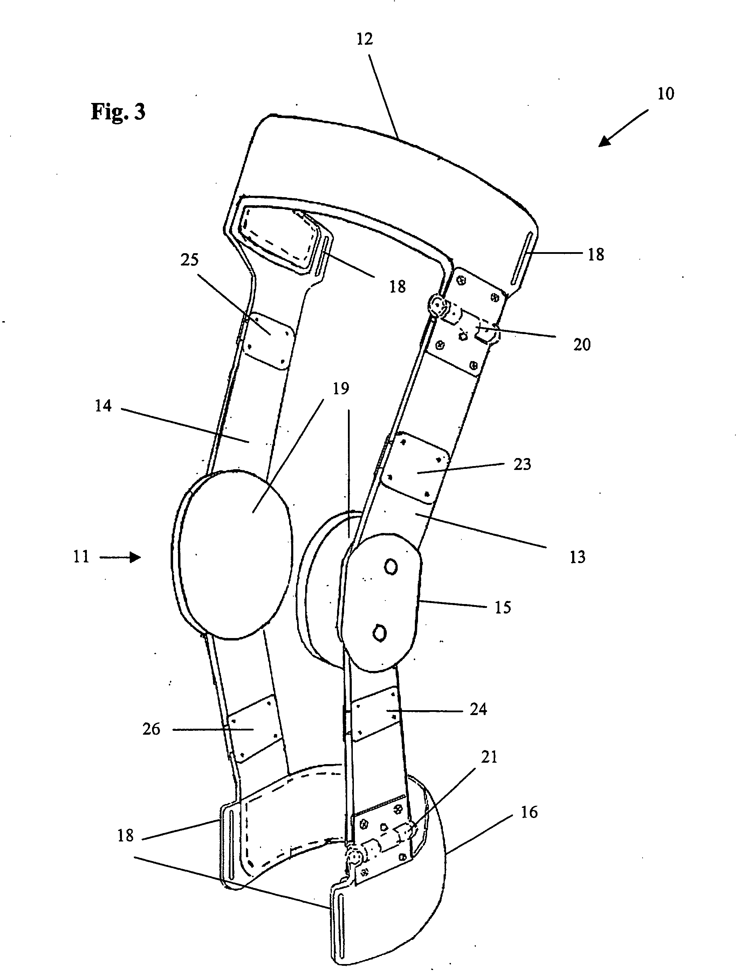

According to a particular embodiment of this invention shown in figure 3 the brace 10 comprises a fixed frame 11 consisting of at least one upper arch 12 integral with a pair of elastically deformable side uprights 13, 14, with which it forms a single body.

The elastically deformable side uprights 13, 14 are equipped with respective joints 15 in their central part.

The joints 15 consist of two toothed wheel portions designed to engage with each other and each one respectively forming a single body with the upper and lower part of the upright.

These toothed wheel portions rotate on two pins and are kept in place by metal plates fitted on opposite sides.

In this way the joint 15 is rigid, not allowing any movement of its components in the normal direction of the axis of the brace.

The side uprights 13, 14 are designed to connect the upper arch 12 with at least one lower arch 16 positioned at the opposite longitudinal end of the brace 10.

According to a particular embodiment of the present invention, the arches 12 and 16 can be positioned on opposite sides of the elastically deformable uprights 13, 14 with for example the arch 12 on the front part of the limb and the arch 16 on the rear part.

In correspondence with each arch 12 and 16 and on opposite sides to the arches, each elastically deformable side upright 13, 14 presents a pair of slots 18 designed to accommodate means of restraining the brace 10 on the user's limb.

The restraining means can consist of straps (not shown in the figures) with gripping adjustment achieved for example by means of the respective Velcro® fixing devices.

Alternatively, the fixing device for each strap can be the pull-off type, consisting of a pair of surfaces, in composite plastic material, with a plurality of reciprocally engaging micropins according to the background art.

The rigid joints 15 present a condylar plate 19 designed to come into contact with the knee joint, to uniformly transmit the force exercised by the elastically deformable side upright to the knee joint and to contain and support the knee joint.

According to the embodiment of this invention shown in figure 3 the elastically deformable side upright 13 presents two hinges 20, 21 with angular micrometric adjustment and two elastic and/or flexible elements 23, 24 forming a single body with the upright 13; a first hinge 20 is positioned above the joint and connected by a wing to the upper arch 12 and by another wing to a first elastic element 23.

The first elastic element 23 is in turn connected on the upright 13 on one side to the first hinge 20 and on the other side to the joint 15.

A second hinge 21 is positioned below the joint 15, connected with one wing to the lower arch 16 and with another wing to a second elastic element 24.

The second elastic element 24 is in turn connected in one direction to the second hinge 21 and in the other direction to the joint 15.

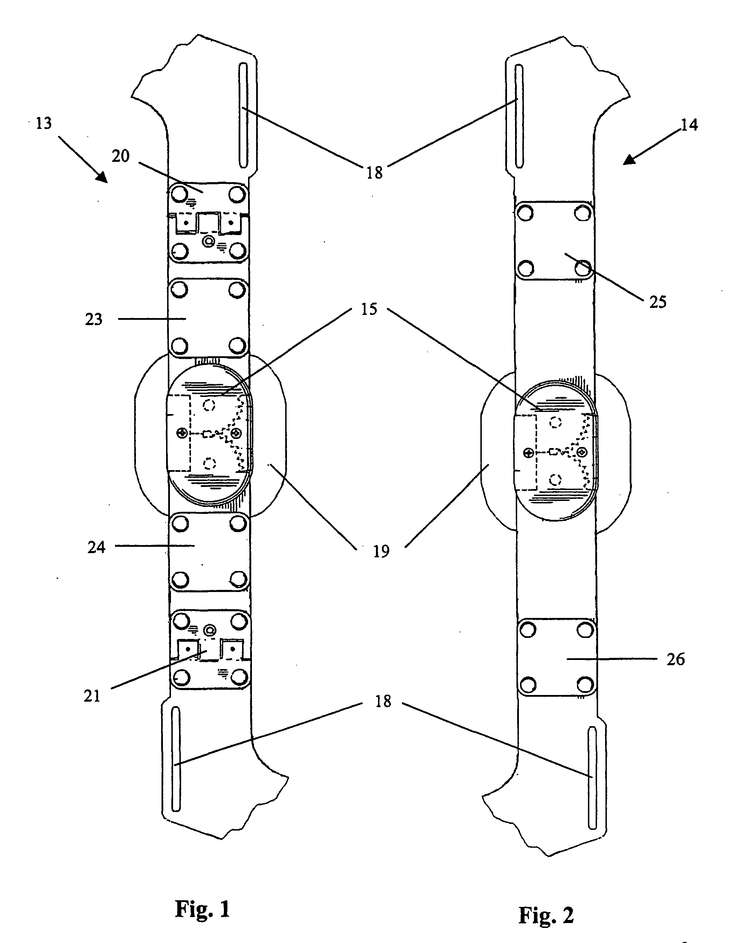

The side upright 13 as described above is shown in detail in figure 1.

The second flexible side upright of the rigid frame according to the particular embodiment shown in figure 3, and as represented in detail in figure 2, presents two elastic elements 25, 26 forming a single body with the second elastically deformable side upright 14; a first elastic element 25 is positioned above the joint 15 and is connected in one direction to the upper arch 12 and in the other direction to the rigid joint 15.

A second elastic element 26 of the second upright 14 is positioned below the joint 15 and is connected in one direction to the lower arch 16 and in the other direction to the rigid joint 15.

According to this invention, the angular flexion of these hinges 20, 21 in cooperation with the elastic elements 23, 24 transmits a force perpendicular to the axis of this elastically deformable side upright 13 in correspondence with the rigid joint 15 to the condylar plate 19.

The pressure obtained at the level of the knee joint alters the contact between the opposite condyles of the knee bone, reducing the pressure on them and thus limiting the cause of inflammation and the source of pain.

According to a particular embodiment of this invention the brace 10 can present a single elastically deformable side upright 14 which can comprise just one hinge with angular micrometric adjustment positioned in one of its parts, above or below the rigid joint 15 and one elastic element in the other part.

A lighter structure is thus obtained which nevertheless performs the lateral pressure action on the knee joint and provides acceptable control of the limb.

According to a particular embodiment of the invention the rigid joints 15 present stops designed to limit the relative angular excursion of the side uprights 13, 14 in order to prevent hyperextension of the knee which can cause pain.

The fixed frame 11 can be made from aluminium, atoxic stainless steel, metal alloys or highly resistant composite plastic material to make the structure lighter.

The elastic elements 23, 24, 25, 26, inserted in the uprights 13, 14 can be steel foils or parts made from highly resistant and elastic plastic material.

The straps constituting the restraining elements of the brace can be made from fabric, while the arches 12 and 16 can be made from highly resistant composite plastic material or atoxic stainless steel forming, if necessary, a single body with the respective side uprights.

According to a particularly advantageous embodiment of the invention the side uprights and the upper and lower arches can be separate elements of different sizes designed to be connected together to form the structure 11 of the brace 10 according to the individual dimensions of the limb and of the respective knee joint.

The invention is described with reference to a preferred embodiment.

## Claims

1. A knee brace or orthesis (10) which presents a frame (11) consisting of at least one upper arch (12) and one lower arch (16) integral with at least one side upright (13) with which they can form a single body, and means of restraining this fixed frame to the upper and lower parts of the limb, this upright (13) being equipped in its central part with a joint (15) consisting of components which are not permitted to move in the normal direction of the axis of the brace, wherein at least one of the parts, upper or lower with respect to the joint (15), of the side upright (13) comprises at least one hinge (20, 21) with angular micrometric adjustement **characterised in that** said at least one of the parts of the side upright (13) is elastically deformable, said at least one hinge being designed to cooperate with the elastic portion of the side upright (13) in order to transmit a force perpendicular to the axis of the upright in correspondence with the rigid joint (15).

2. A brace according to claim 1, **characterised in that** the joint (15) comprises two toothed wheel portions designed to engage with each other and each constituting a single body respectively with the upper and lower part of the upright, said toothed wheel portions rotating on two pins and being rigidly kept in place by two opposite metal plates.

3. A brace (10) according to claim 1 or 2, **characterised in that** the fixed frame (11) is equipped with a second side upright (14) at least one part of which, upper or lower with respect to the central joint (15), is integral with the upper (12) and lower arch (16) of the structure (11) on the other side with respect to the first elastically deformable upright (13), in such a way that the transversal pressure exercised on the knee joint by the first elastically deformable upright (13) is dispersed with the elastic deformation of the second upright (14) without causing compression of the knee joint.

4. A brace according to any of the preceding claims **characterised in that** it comprises elastic and/or flexible elements (23, 24, 25, 26) which connect to form a single body with the upper and lower parts of the uprights (13, 14) with respect to the joints (15).

5. A brace according to claim 3 **characterised in that** said elastic and/or flexible elements (23, 24, 25, 26) consist of steel foils or segments in composite plastic material.

6. A brace according to any of the preceding claims **characterised in that** the fixed frame (11) presents two elastically deformable side uprights (13, 14) of which a first side upright (13) comprises two hinges with angular micrometric adjustment (20, 21) and two elastic and/or flexible elements (23, 24) forming a single body with the said first upright (13), a first hinge (20) being positioned above the joint (15) and connected by a wing to the upper arch (12) and by another wing to a first elastic element (23), said first elastic element (23) in turn connected to the first upright (13) in the direction of the first hinge (20) and in the other direction to the joint (15), and a second hinge (21) positioned below the joint (15) connected by a wing to the lower arch (16) and by another wing to a second elastic element (24), said second elastic element (24) in turn connected in one direction to the second hinge (21) and in the other direction to the joint (15), and a second elastically deformable side upright (14) comprising two elastic elements (25, 26) forming a single body with the second upright (14), a first elastic element (25) of the second upright (14) being positioned above the joint (15) and connected in one direction to the upper arch (12) and in the other direction to the joint (15) and a second elastic element (26) of the second upright (14) being positioned below the joint (15) and connected in one direction to the lower arch (16) and in the other direction to the joint (15).

7. A brace (10) according to any of the preceding claims, **characterised in that** said fixed frame (11) comprises at least one upper arch (12) and at least one lower arch (16), which when worn are turned towards the rear part of the limb or on opposite sides to each other, connected or forming a single body with the elastically deformable side uprights (13, 14) interrupted in their central section by the joints (15).

8. A brace (10) according to any of the preceding claims, **characterised in that** the upper arch (12), the lower arch (16) and the elastically deformable side uprights (13, 14) are separate elements that can be connected together and can be of different dimensions and sizes.

9. A brace (10) according to any of the preceding claims, **characterised in that** said restraining means of the frame to the femur and the tibia connected for example to the slots (18) consist of straps, fitted with variable extension gripping means.

10. A brace (10) according to claim 8, **characterised in that** said gripping means consist of pull-off fastenings.

11. A brace (10) according to claim 8, **characterised in that** said gripping means consist of pull-off fastenings comprising a pair of surfaces, in composite plastic material, with a plurality of reciprocally engaging micropins.

12. A brace (10) according to any of the preceding claims, **characterised in that** said joints (15) present stops designed to limit the relative angular excursion of said side uprights (13, 14).

13. A brace (10) according to any of the preceding claims, **characterised in that** said joints (15) present a condylar plate (16) designed to enter into contact with the knee joint, uniformly transmitting the force exercised by one elastically deformable side upright (13) to the knee joint and to contain and support said knee joint.

14. A brace (10) according to any of the preceding claims, **characterised in that** said elastic elements (23, 24, 25, 26) consist for example of steel foils or composite plastic material.

15. A brace (10) according to any of the preceding claims, **characterised in that** said fixed frame (11) is made from atoxic stainless steel, metal alloys or highly resistant composite plastic material to make the structure lighter.

## Patentansprüche

1. Knieschiene oder -orthese (10), die einen Rahmen (11) aufweist, der aus wenigstens einem oberen Bogen (12) und einem unteren Bogen (16) besteht, die mit wenigstens einem seitlichen Vertikalstab (13) integriert ausgebildet sind, mit dem sie einen einzelnen Körper bilden können, sowie Mittel zum Befestigen dieses festen Rahmens an dem oberen und dem unteren Teil des Beines, wobei der Vertikalstab (13) an seinem mittleren Teil mit einem Gelenk (15) ausgestattet ist, das aus Teilen besteht, die sich nicht senkrecht zu der Achse der Schiene bewegen dürfen, wobei wenigstens eines der Teile, das obere oder das untere bezogen auf das Gelenk (15) des seitlichen Vertikalstabs (13) wenigstens ein Scharnier (20, 21) mit einer Mikrometerwinkeleinstellung besitzt, **dadurch gekennzeichnet, dass** wenigstens eines der Teile des seitlichen Vertikalstabs (13), der elastisch verformbar ist, dazu ausgebildet ist, mit dem elastischen Abschnitt des seitlichen Vertikalstabs (13) zusammenzuwirken, um eine Kraft, die senkrecht zu der Achse des Vertikalstabs zusammenwirkend mit dem starren Gelenk (15) zu übertragen.

2. Schiene nach Anspruch 1, **dadurch gekennzeichnet, dass** das Gelenk (15) aus zwei Zahnradabschnitten besteht, die so ausgebildet sind, dass sie ineinander eingreifen und jeweils einteilig mit dem oberen und unteren Teil des Vertikalstabs ausgebildet sind, wobei die Zahnradabschnitte sich auf zwei Zapfen drehen und durch zwei einander gegenüberliegende Metallplatten starr an Ort und Stelle gehalten werden.

3. Schiene (10) nach Anspruch 1 oder 2, **dadurch gekennzeichnet, dass** der feste Rahmen (11) mit einem zweiten seitlichen Vertikalstab (14) ausgestattet ist, von dem wenigstens ein Teil, das obere oder das untere bezüglich des mittleren Gelenks (15), mit dem oberen (12) und unteren (16) Bogen der Struktur auf der in Bezug auf den ersten elastisch deformierbaren Vertikalstab (13) integriert ausgeführt ist, so das der durch den ersten elastisch deformierbaren Vertikalstab (13) auf das Kniegelenk ausgeübte Querdruck durch die elastische Deformation des zweiten Vertikalstabs (14) verteilt wird, ohne einen Druck auf das Kniegelenk zu bewirken.

4. Schiene nach einem der vorhergehenden Ansprüche, **dadurch gekennzeichnet, dass** sie elastische und/oder flexible Elemente (23, 24, 25, 26) besitzt, die mit den oberen und unteren Teilen der Vertikalstäbe (13, 14) bezogen auf die Gelenke (15) einstückig verbunden sind.

5. Schiene nach Anspruch 3, **dadurch gekennzeichnet, dass** die elastischen und/oder flexiblen Elemente (23, 24, 25, 26) aus Stahlblechen oder Segmenten aus Verbundkunststoffmaterial bestehen.

6. Schiene nach einem der vorhergehenden Ansprüche, **dadurch gekennzeichnet, dass** der feste Rahmen (11) zwei elastisch deformierbare seitliche Vertikalstäbe (13, 14) aufweist, von denen ein erster seitlicher Vertikalstab (13) zwei Scharniere mit Mikrometerwinkeleinstellungen (20, 21) besitzt, sowie zwei elastische und/oder flexible Elemente (23, 24), die mit dem ersten Vertikalstab (13) einstückig sind, wobei ein erstes Scharnier (20) über dem Gelenk (15) angeordnet und durch einen Flügel mit dem oberen Bogen (12) und durch einen anderen Flügel mit einem ersten elastischen Element (23) verbunden ist, wobei das erste elastische Element (23) seinerseits in Richtung des ersten Scharniers (20) mit dem ersten Vertikalstab (13) verbunden ist und in der anderen Richtung mit dem Gelenk (15), und wobei ein zweites Scharnier (21), das unterhalb des Gelenks (15) angeordnet ist, mit einem Flügel mit dem unteren Bogen (16) und mit dem anderen Flügel mit einem zweiten elastischen Element (24) verbunden ist, das wiederum in einer Richtung mit dem zweiten Scharnier (21) und in der anderen Richtung mit dem Gelenk verbunden ist, und wobei ein zweiter elastisch deformierbarer seitlicher Vertikalstab (14) zwei elastische Elemente (25, 26) besitzt, die einstückig mit dem zweiten Vertikalstab (14) ausgebildet sind, wobei ein erstes elastisches Element (25) des zweiten Vertikalstabs (14) über dem Gelenk (15) angeordnet und in einer Richtung mit dem oberen Bogen (12) und in der anderen Richtung mit dem Gelenk (15) verbunden ist und wobei ein zweites elastisches Element (26) der zweiten Vertikalstütze (14) unterhalb des Gelenks (15) angeordnet und in einer Richtung mit dem unteren Bogen (16) und in der anderen Richtung mit dem Gelenk (15) verbunden ist.

7. Schiene (10) nach einem der vorhergehenden Ansprüche, **dadurch gekennzeichnet, dass** der feste Rahmen (11) aus wenigstens einem oberen Bogen (12) und wenigstens einem unteren Bogen (16) besteht, die beim Tragen in Richtung auf die Rückseite des Beines oder in entgegen gesetzter Richtung zueinander gedreht sind, und die mit den elastisch deformierbaren seitlichen Vertikalstützen, die in ihren mittleren Abschnitten durch die Gelenke (15) unterbrochen sind, verbunden oder einstückig sind.

8. Schiene (10) nach einem der vorhergehenden Ansprüche, **dadurch gekennzeichnet, dass** der obere Bogen (12), der untere Bogen (16) und die elastisch deformierbaren seitlichen Vertikalstützen (13 14) einzelne Elemente sind, die miteinander verbunden werden können und die unterschiedliche Dimensionen und Größen aufweisen können.

9. Schiene (10) nach einem der vorhergehenden Ansprüche, **dadurch gekennzeichnet, dass** die Befestigungsmittel des Rahmens an dem Femur und der Tibia, die beispielsweise mit den Schlitzen (18) verbunden sind, aus Gurten bestehen, die mit Haltemitteln mit variabler Länge ausgestattet sind.

10. Schiene (10) nach Anspruch 9, **dadurch gekennzeichnet dass** die Haltemittel aus Klettverschlüssen bestehen.

11. Schiene (10) nach Anspruch 9, **dadurch gekennzeichnet, dass** die Haltemittel aus Klettverschlüssen bestehen, die aus einem Flächenpaar aus Verbundkunststoff bestehen, die eine Mehrzahl von miteinander in Eingriff kommenden Mikrozapfen aufweisen.

12. Schiene (10) nach einem der vorhergehenden Ansprüche **dadurch gekennzeichnet, dass** die Gelenke (15) Anschläge besitzen, die dazu ausgelegt sind, den relativen Winkelausschlag der seitlichen Vertikalstützen (13, 14) zu begrenzen.

13. Schiene (10) nach einem der vorhergehenden Ansprüche, **dadurch gekennzeichnet, dass** die Gelenke (15) eine Kondylenplatte (16) besitzen, die dazu ausgelegt ist, mit dem Kniegelenk in Kontakt zu kommen und die die Kraft, die durch eine elastisch deformierbare seitliche Vertikalstütze (13) auf das Kniegelenk ausgeübt wird, gleichmäßig überträgt und die das Kniegelenk umschließt und stützt.

14. Schiene (10) nach einem der vorhergehenden Ansprüche, **dadurch gekennzeichnet, dass** die elastischen Elemente (23, 24, 25, 26) beispielsweise aus Stahlblech oder Kunststoffverbundmaterial bestehen.

15. Schiene (10) nach einem der vorhergehenden Ansprüche, **dadurch gekennzeichnet, dass** der feste Rahmen (11) aus einem ungiftigen rostfreien Stahl, Metalllegierungen oder, um die Struktur leichter zu machen, einem sehr widerstandsfähigen Kunststoffverbundmaterial besteht.

## Revendications

1. Appareil orthopédique pour genou ou orthèse (10) présentant un cadre (11) composé d'au moins un cintre supérieur (12) et d'un cintre inférieur (16) solidaires d'au moins un montant latéral (13) avec lequel ils peuvent constituer une seule pièce, et des moyens pour maintenir ce cadre fixe aux parties supérieure et inférieure du membre, ce montant (13) étant équipé en sa partie centrale d'un joint (15) constitué de composants qui ne pouvent pas bouger dans le sens normal de l'axe de l'appareil orthopédique, dans lequel au moins l'une des parties, supérieure ou inférieure par rapport au joint (15), du montant latéral (13) comprend au moins une charnière (20, 21) avec un réglage micrométrique angulaire, **caractérisé en ce qu'**au moins l'une desdites parties du montant latéral (13) est déformable élastiquement, au moins l'une desdites charnières étant conçue pour coopérer avec la portion élastique du montant latéral (13) pour transmettre une force perpendiculaire à l'axe du montant en fonction du joint rigide (15).

2. Appareil orthopédique selon la revendication 1, **caractérisé en ce que** le joint (15) comprend deux portions à roue dentée conçues pour s'engager l'une dans l'autre et chacune constituant une seule pièce respectivement avec les parties supérieure et inférieure du montant, lesdites portions à roue dentée tournant sur deux pivots et étant maintenues solidement en place par deux plaques métalliques opposées.

3. Appareil orthopédique (10) selon la revendication 1 ou 2, **caractérisé en ce que** le cadre fixe (11) est équipé d'un second montant latéral (14) dont au moins l'une des parties, supérieure et inférieure par rapport au joint central (15), est solidaire du cintre supérieur (12) et du cintre inférieur (16) de la structure (11) de l'autre côté par rapport au premier montant déformable élastiquement (13), de telle sorte que la pression transversale exercée sur l'articulation du genou par le premier montant déformable élastiquement (13) est dispersée avec la déformation élastique du second montant (14) sans provoquer la compression de l'articulation du genou,

4. Appareil orthopédique selon l'une quelconque des revendications précédentes, **caractérisé en ce qu'**il comprend des éléments élastiques et/ou flexibles (23, 24, 25, 26) qui se connectent pour constituer une seule pièce avec les parties supérieure et inférieure des montants (13, 14) par rapport aux joints (15).

5. Appareil orthopédique selon la revendication 3, **caractérisé en ce que** lesdits éléments élastiques et/ou flexibles (23, 24, 25, 26) se composent de films d'acier ou de segments en matériau plastique composite.

6. Appareil orthopédique selon l'une quelconque des revendications précédentes, **caractérisé en ce que** le cadre fixe (11) présente deux montants latéraux déformables élastiquement (13, 14) dont un premier montant latéral (13) comprend deux charnières avec un réglage micrométrique angulaire (20, 21) et deux éléments élastiques et/ou flexibles (23, 24) formant une seule pièce avec ledit premier montant (13), une première charnière (20) étant disposée au-dessus du joint (15) et connectée par une aile au cintre supérieur (12) et par une autre aile à un premier élément élastique (23), ledit premier élément élastique (23) étant connecté à son tour au premier montant (13) dans le sens de la première charnière (20) et dans l'autre sens au joint (15), et une seconde charnière (21) étant disposée au-dessous du joint (15) et connectée par une aile au cintre inférieur (16) et par une autre aile à un second élément élastique (24), ledit second élément élastique (24) étant à son tour connecté dans un sens à la seconde charnière (21) et dans l'autre sens au joint (15), et un second montant latéral déformable élastiquement (14) comprenant deux éléments élastiques (25, 26) formant une seule pièce avec le second montant (14), un premier élément élastique (25) du second montant (14) étant disposé au-dessus du joint (15) et connecté dans un sens au cintre supérieur (12) et dans l'autre sens au joint (15) et un second élément élastique (26) du second montant (14) étant disposé au-dessous du joint (15) et connecté dans un sens au cintre inférieur (16) et dans l'autre sens au joint (15).

7. Appareil orthopédique (10) selon l'une quelconque des revendications précédentes, **caractérisé en ce que** ledit cadre fixe (11) comprend au moins un cintre supérieur (12) et au moins un cintre inférieur (16), qui une fois portés sont tournés vers la partie arrière du membre ou sur des faces opposées l'une de l'autre, connectés ou formant une seule pièce avec les montants latéraux déformables élastiquement (13, 14) interrompus en partie centrale par les joints (15).

8. Appareil orthopédique (10) selon l'une quelconque des revendications précédentes, **caractérisé en ce que** le cintre supérieur (12), le cintre inférieur (16) et les montants latéraux déformables élastiquement (13, 14) sont des éléments séparés susceptibles de se connecter l'un à l'autre et d'être de dimensions et de tailles différentes.

9. Appareil orthopédique (10) selon l'une quelconque des revendications précédentes, **caractérisé en ce que** lesdits moyens pour maintenir le cadre contre le fémur et le tibia,, connectés par exemple aux fentes (18), se composent de bandes, équipées de moyens d'accrochage d'extension variable.

10. Appareil orthopédique (10) selon la revendication 8, **caractérisé en ce que** lesdits moyens d'accrochage se composent de fixations arrachables.

11. Appareil orthopédique (10) selon la revendication 8, **caractérisé en ce que** lesdits moyens d'accrochage se composent de fixations arrachables comprenant une paire de surfaces, en matériau plastique composite, avec une pluralité de micro-ergots s'engageant l'un dans l'autre.

12. Appareil orthopédique (10) selon l'une quelconque des revendications précédentes, **caractérisé en ce que** lesdits joints (15) présentent des butées conçues pour limiter le déplacement angulaire relatif desdits montants latéraux (13, 14).

13. Appareil orthopédique (10) selon l'une quelconque des revendications précédentes, **caractérisé en ce que** lesdits joints (15) présentent une plaque condylienne (16) conçue pour venir au contact de l'articulation du genou, transmettant de manière uniforme la force exercée par un montant latéral déformable élastiquement (13) à l'articulation du genou et pour maintenir et soutenir ladite articulation du genou.

14. Appareil orthopédique (10) selon l'une quelconque des revendications précédentes, **caractérisé en ce que** lesdits éléments élastiques (23, 24, 25, 26) se composent par exemple de films d'acier ou de matériau plastique composite.

15. Appareil orthopédique (10) selon l'une quelconque des revendications précédentes, **caractérisé en ce que** ledit cadre fixe (11) est réalisé en acier inoxydable atoxique, alliages de métaux ou matériau plastique composite extrêmement résistant pour alléger la structure.
